# EUROPEAN PATENT APPLICATION

(11) **EP 2 189 103 A1**
(43) Date of publication of application: **26.05.2010**
(21) Application number: 08830584.2
(22) Date of filing: 11.09.2008
(51) Int. Cl.: A61B 1/00, G02B 23/24

(54) **ENDOSCOPE DEVICE**

(30) Priority: 11.09.2007 JP 2007235475
(71) Applicant: OLYMPUS CORPORATION, Shibuya-ku Tokyo 151-0072 (JP)
(72) Inventor: KONOMURA, Yutaka, Tokyo 151-0072 (JP)
(74) Representative: Gunzelmann, Rainer
(86) International application number: PCT/JP2008/066454
(87) International publication number: WO 2009/035051

(57) **Abstract**

An operation portion of an endoscope includes: an insertion-portion-side housing (12) including an insertion portion 2 having a bending portion; a main-unit-side housing (13); and an operation lever (14) for operating a bending portion. The insertion-portion-side housing includes: wires (20a to 20d) for bending the bending portion; first engagement members (19a to 19d) capable of moving forward and backward in their longitudinal direction; and coil springs (24a to 24d). The main-unit-side housing includes: operation wires (29a to 29d) connected to the operation lever; second engagement members (27a to 27d) that are connected to the operation wires and are capable of moving in their longitudinal direction and capable of engaging the first engagement members; and coil springs (31a to 31d). When the insertion-portion-side housing and the main-unit-side housing are slid and coupled, the first engagement members and the second engagement member are respectively brought into engagement with each other, to thereby transmit an actuation of the operation lever to the bending portion of the insertion portion. According to the present invention, it is possible to replace an insertion-portion-side housing without aligning with the main-unit-side housing.

## Description

### TECHNICAL FIELD

The present invention relates to an endoscope apparatus for bendingly actuating an insertion portion of an endoscope, other treatment tools, or the like.
Priority is claimed on Japanese Patent Application No. 2007-235475, filed on September 11, 2007, the contents of which are incorporated herein by reference.

### BACKGROUND ART

Conventional apparatuses for replacing an insertion portion of an endoscope, other treatment tools, or the like include ones described in, for example, Patent Documents 1 and 2. In these apparatuses, a plurality of wire sets in which each set consists of two wires are connected to a bending portion of an insertion portion. The wires in each set are pulled and relaxed, to thereby bend the bending portion at the distal end in the left-right direction, the up-down direction, and other directions. Such a distal-end portion of an endoscope or the like including an insertion portion can be separated for replacement from the main unit at the connection part, and another type of distal end portion can be attached to the main unit.
In the treatment tool described in Patent Document 1, the distal-end portion is capable of being separably replaced at its connection portion with the main unit portion.
In connecting the distal-end portion with the main unit portion, a plurality of step-like teeth portions, which are provided to each of the two wires in each set at their connection part, are brought into mesh with each other. In this case, incorrect mesh position results in inconveniences as follows.
That is, if the teeth portions are brought into mesh while one or both of the two wires are relaxed, an operation on the operation portion to bend the bending portion at the distal end of the insertion portion does not lead to an immediate bending actuation due to the slack in the wire(s), and as a result, operability is lowered. Furthermore, even if the teeth portions are brought into mesh with each other while the two wires are in a tense state without a slack, and also the angle operation system is in its neutral position, the center position for a bending operation of the bending portion at the distal end of the insertion portion is displaced when the bending portion is a bent state. In addition, a further bending operation in this condition applies excessive bend on the insertion portion.

To avoid such inconveniences, when separating the distal end portion from the main unit portion in a first connective coupling construction, the treatment tool according to Patent Document 1 retains one of the step-like teeth portions, which function as a connection part between the two, in abutment with the positioning member at the proximal end. Therefore, when another distal-end portion is coupled to the main unit portion at the connection part, the position and the tension of the wires before the separation are reproducible.
Furthermore, as a second connective coupling construction of the treatment tool, there is proposed a method of detecting a neutral position of the wires subsequent to removal of play by moving the wires after connecting the teeth portions with each other. Consequently, it is required to provide a sensor for detecting the positions of the wires.
As a third connective coupling construction of the treatment tool, there is disclosed a construction in which the wires are connected by meshing a single teeth portion of one teeth portion with a single teeth portion of the other. In this treatment tool, a member with a first teeth portion is inserted into a box containing a second teeth portion. Then, the first teeth portion is lifted in the box, to thereby bring the teeth portions into mesh with each other.

In the endoscope described in Patent Document 2, a bending drive portion for a bending actuation of a bending portion at a distal end of the insertion portion is made of a separable gearbox and a bend-pulling mechanism portion. The bend-pulling mechanism portion has a sprocket for bending wires and a rotation shaft. In the gearbox, there are provided a motor for driving the rotation shaft and an encoder. In this construction, it is not possible to find the positions of the wires at the time of its attachment/detachment. Therefore, the encoder is used to detect the wire positions.
Patent Document 1: PCT International Patent Publication No. WO 2006/053198 pamphlet Patent Document 2: Japanese Unexamined Patent Application, First Publication No. 2004-121414

However, in the first connective coupling construction in Patent Document 1, the adoption of such a construction in an endoscope moves the two wires in the sets for bending the distal end of the endoscope in the mutually opposite directions. Therefore, one wire is movable in the forward-backward direction, however, the other wire has its teeth portion in abutment with the positioning member, and hence is unable to move backward further than the position of abutment. Consequently, it is not possible to adopt such a connective coupling construction in an endoscope.
In the second connective coupling construction, it is required to provide a sensor for detecting the positions of the wires in the distal-end portion of the treatment tool, and to move, after the connection, the wires one by one for removal of a slack, to thereby detect the correct positions of the wires. This results in complexity in construction and adjustment, and also results in a high manufacturing cost. Furthermore, in the third connective coupling construction, no technique for retaining the teeth portions in mesh with each other is disclosed. Therefore, it is not certain whether this construction is adoptable in an endoscope.
Furthermore, in the construction of the endoscope described in Patent Document 2, it is configured such that an encoder is used to detect the wire positions because it is not possible to find the wires position at the time of attachment/detachment. This results not only in a high manufacturing cost of the bend-pulling mechanism portion in the member to be detached, but also in a larger size of its construction.

### DISCLOSURE OF INVENTION

In view of such circumstances, the present invention has an object to provide an endoscope apparatus in which an insertion-portion-side housing including an insertion portion with a bending portion is capable of being detachably replaced with ease without positioning.

An endoscope apparatus according to the present invention includes: a first portion that communicates with an insertion portion having a bendable bending portion; a second portion that is detachably attached to the first portion; and a drive portion that is provided on the second portion side and bendingly moves the bending portion. The first portion includes: a first pulling member that is connected to the bending portion and bendingly moves the bending portion; and a first engagement member that is connected to the first pulling member and is capable of moving forward and backward in a longitudinal direction of the first pulling member. The second portion includes: an actuation member that is connected to the drive portion; and a second engagement member that is connected to the actuation member, and is capable of moving forward and backward in the longitudinal direction of the first pulling member and of engaging the first engagement member. Engagement between the first engagement member and the second engagement member transmits an actuation of the drive portion to the bending portion of the insertion portion.
According to the present invention, for example, the first engagement member and the second engagement member are relatively slid to engage the first engagement member provided in the first portion with the second engagement member provided in the second portion. In this state, actuation of the drive portion transmits the actuation from the actuation member to the first pulling member via the second engagement member and the first engagement member, to thereby make it possible to bend the bending portion of the insertion portion in an appropriate direction. In addition, in engaging the first engagement member with the second engagement member, the necessity of previous positioning of the two is eliminated, enabling their engagement at an optional position. Therefore, in performing an observation, treatment, or the like of a body cavity, a mechanical structure, or the like by use of the insertion portion of the endoscope apparatus, it is possible, as required, to remove the insertion portion together with the first portion for replacement and attach another first portion to the second portion with ease.

In the endoscope apparatus, it is preferable that a positioning-fixation mechanism that positions and fixes the first portion and the second portion be provided and that positioning and fixing the first portion and the second portion by the positioning-fixation mechanism engage the first engagement member with the second engagement member.
With the positioning of the first portion and the second portion by the positioning-fixation mechanism, it is possible to set the first engagement member and the second engagement member securely in an engaged state.
The endoscope apparatus may include one or more wires as the first pulling member. In this case, it is possible to bend the insertion portion in one or more directions in accordance with the number of the wires.
The endoscope apparatus may include one or more pairs of wires that move in mutually opposite directions as the first pulling member.
With the mutual movement of one or more pairs of wires, it is possible to bend the insertion portion in an appropriate direction.

The first portion may include a first biasing member that connects the first engagement member to a side opposite to the first pulling member in a biased state, and the second portion may include a second biasing member that connects the second engagement member to a side opposite to the actuation member in a biased state, the actuation member being a second pulling member.
With the first and second biasing members, it is possible to prevent unnecessary movement of movable parts such as the first engagement member and the second engagement member. In coupling the first portion to the second portion, the bending portion of the insertion portion is capable of bending in the mutually opposite directions. Therefore, in the pair of the first and second engagement members, if one is at its moved-forth position, then the other is at its moved-back position. Accordingly, even if either one of the first and second engagement members at its moved-back position does not abut the other of the first and second engagement members, the other is brought into abutment with one because the other is at its moved-forth position. Furthermore, the position of the other is adjusted by the biasing forces of the first and second biasing members. As a result, the aforementioned one of the first and second engagement members is moved and brought into abutment. Therefore, it is possible to hold the first and second engagement members at balanced positions with the adjustment of their moved-forth and moved-back positions by the biasing forces of the first and second biasing members.
The actuation member may be a second pulling member, and the drive portion may be an operation lever to which the second pulling member is connected.
Operating the operation lever actuates the second engagement member via the second pulling member, to thereby cause the first engagement member which the second engagement member engages to interlockedly move. This makes it possible to appropriately bend the bending portion of the insertion portion via the first pulling member.

The first portion may include a second biasing member that connects the second engagement member to an opposite side of the operation member in a biased state, and the actuation member may be a second pulling member.
Even if the second engagement member is at any position such as its moved-forth position or moved-back position when the insertion-portion-side housing is attached to the main-unit-side housing, it is possible to adjust the position of the first engagement member based on the position of the second engagement member with the biasing force applied by the first biasing member when the first engagement member is brought into abutment and engaged with the second engagement member. This enables the alignment or positioning between the insertion-portion-side housing and the main-unit-side housing. At this time, a space may be provided between the first engagement member and the second engagement member by the second engagement member being moved back.
Then, the direct drive mechanism is interlockedly operated by the drive portion to adjust the forward and backward movement of the second engagement member. This makes it possible to adjust the second engagement member into a neutral state where it is in engagement with the first engagement member. Furthermore, with the actuation of the drive portion, the second engagement member and the first engagement member are moved forward or backward via a screw member. This makes it possible to bend the bending portion of the insertion portion to an appropriate position via the first pulling member.

The drive portion may be a motor that rotationally actuates the direct drive mechanism to interlockedly move the second engagement member.
In bending the bending portion of the insertion portion, the direct drive mechanism is rotated by the motor drive to move the second engagement member forward and backward. This causes the first engagement member to interlockedly move in a state engaged with the second engagement member. Thereby, it is possible to bendingly operate the bending portion via the first pulling member.
It is preferable that the first engagement member and the second engagement member each include a step portion, in which the step portions are capable of engaging and detaching from each other, at least either one of the two being slidable along a guide groove.
With the engagement between the first engagement member and the second engagement member at their step portions, the forward and backward movement of the second engagement member can be transmitted to the first engagement member. Furthermore, the bending portion of the insertion portion is capable of bending in the mutually opposite directions. Therefore, the forward and backward movement of one or both of the step portions along the guide groove makes it possible to adjust the positions of the pair of the engaged first engagement member and second engagement member in the opposite forward and backward directions.

It is preferable that a guide member be provided in one of the first portion and the second portion, and that an anchor groove along which the guide member is slidable is provided in the other, and that the first engagement member and the second engagement member be configured to be brought into engagement with each other in a state with the guide member in engagement with the anchor groove.
The guide member and the anchor groove are relatively slidable. Therefore, for example, it is possible to put the first engagement member and the second engagement member in an engaged state at the same time when the first portion and the second portion are relatively slid and coupled.
The positioning-fixation mechanism may include: a lock member that fixes the first portion and the second portion in a coupled state; and a release member that releases locking by the lock member.
With the lock member and the release member of the positioning-fixation mechanism, it is possible to perform a coupling and releasing operation between the first portion and the second portion.
Note that the attachment and release by the lock member and the release member may be in conjunction with the sliding between the guide member and the anchor groove.

The present invention is an endoscope apparatus that includes an insertion portion having an observation system and an illumination system, including: a first portion that communicates with an insertion portion having a bendable bending portion; a second portion that is detachably attached to the first portion; and an operation device that is provided in the second portion and bendingly moves the bending portion. The first portion includes: a first pulling member that is connected to the bending portion and bendingly moves the bending portion; and a first engagement member that is connected to the first pulling member and is capable of moving forward and backward in a longitudinal direction of the first pulling member. The second portion includes: a second pulling member that is connected to the operation device; and a second engagement member that is connected to the second pulling member, and is capable of moving forward and backward in the longitudinal direction of the second pulling member and of engaging the first engagement member. Engagement between the first engagement member and the second engagement member as a result of coupling the first portion to the second portion transmits an actuation of the operation device to the bending portion of the insertion portion.
According to the present invention, for example, the first portion and the second portion are relatively slid to engage the first engagement member with the second engagement member. In this state, actuation of the operation device transmits the actuation from the second pulling member to the first pulling member via the second engagement member and the first engagement member, to thereby make it possible to bend the bending portion of the insertion portion in an appropriate direction.
Furthermore, in performing an observation, a treatment, or the like of the interior of a body cavity, a mechanical structure, or the like by use of the insertion portion of the endoscope apparatus according to the present invention, the insertion portion together with the first portion can be removed for replacement as required, and another first portion can be attached to the second portion with ease. Furthermore, at that time, the necessity of previous positioning between the first engagement member and the second engagement member is eliminated.

The present invention is an endoscope apparatus that includes an insertion portion having an observation system and an illumination system, including: a first portion that communicates with an insertion portion having a bendable bending portion; a second portion that is detachably attached to the first portion; and a drive source that is provided on the second portion side and bendingly moves the bending portion. The first portion includes: a first pulling member that is connected to the bending portion and bendingly moves the bending portion; and a first engagement member that is connected to the first pulling member and is capable of moving forward and backward in a longitudinal direction of the first pulling member. The second portion includes: a direct drive mechanism that is connected to the drive source; and a second engagement member that, in response to the direct drive mechanism, is capable of moving forward and backward in the longitudinal direction of the direct drive mechanism and is capable of engaging the first engagement member. Engagement between the first engagement member and the second engagement member as a result of coupling the first portion to the second portion transmits a drive of the drive source to the bending portion of the insertion portion.
According to the present invention, for example, the first portion and the second portion are relatively slid to engage the first engagement member with the second engagement member. In this state, drive of the drive source transmits the actuation from the direct drive mechanism to the first pulling member via the second engagement member and the first engagement member, to thereby make it possible to bend the bending portion of the insertion portion in an appropriate direction. Furthermore, in performing an observation, a treatment, or the like of the interior of a body cavity, a mechanical structure, or the like, the insertion portion together with the first portion can be removed for replacement as required, and another first portion can be attached to the second portion with ease. Furthermore, at that time, the necessity of previous positioning between the first engagement member and the second engagement member is eliminated.

The drive source may be connected to an operation device for actuating the drive source. This allows the drive source to be operated with an operation device such as a remote controller from outside the main-unit-side housing.

According to the endoscope apparatus of the present invention, relative sliding between the first engagement member and the second engagement member allows engagement between the first engagement member and the second engagement member at an optional position. Therefore, when the first portion is attachingly/detachingly replaced, only relative sliding between the first portion and the second portion can easily set the drive portion and the bending portion in an interlockedly operable state at a predetermined position without requiring previous positioning. In addition, the first portion and the second portion are simple in construction, easy in attaching/detaching replacement, and low in their manufacturing cost.

### BRIEF DESCRIPTION OF THE DRAWINGS

FIG. 1 is a diagram showing an entire construction of an endoscope according to a first embodiment of the present invention.
FIG. 2 is an exploded perspective view showing an operation portion of the endoscope according to the first embodiment.
FIG. 3 is a perspective view of an insertion-portion-side housing of the operation portion shown in FIG. 2.
FIG. 4 is a partially enlarged view of a coupling state between the first engagement member and one of the wires shown in FIG. 3.
FIG. 5 is a perspective view of a main-unit-side housing of the operation portion shown in FIG. 2.
FIG. 6 is a partially enlarged view of a coupling state between the second engagement member and the operation wires shown in FIG. 5.
FIG. 7A is a perspective view of the main part that shows an operational construction of an operation lever in the operation portion.
FIG. 7B is an exploded perspective view showing an operational construction of the operation lever in the operation portion.
FIG. 8A is a diagram showing a lock construction between the insertion-portion-side housing and the main-unit-side housing.
FIG. 8B is an enlarged view showing the main part of the lock construction shown in FIG. 8A.
FIG. 9 is a separated state between the insertion-portion-side housing and the main-unit-side housing of the operation portion before they are coupled.
FIG. 10 is a diagram showing a lock construction between the insertion-portion-side housing and the main-unit-side housing of the operation portion.
FIG. 11 is a diagram showing an internal construction of the operation portion in its neutral state.
FIG. 12 is a diagram showing the internal construction of the operation portion when the operation lever is inclined.
FIG. 13 is a diagram showing the internal construction of the operation portion when the operation lever is inclined in the direction opposite to that of FIG. 11.
FIG. 14 is a diagram showing a construction of the main part of an endoscope according to a second embodiment of the present invention.
FIG. 15 is a diagram showing a construction of the main part of the endoscope when an insertion-portion-side housing is removed.
FIG. 16 is a perspective view showing the main part of the main-unit-side housing of the operation portion.
FIG. 17 is a diagram showing second engagement members and threaded screws of the main-unit-side housing.
FIG. 18 is a diagram showing an interlock mechanism of the threaded screws provided with the second engagement members.
FIG. 19 is a diagram showing the interlock mechanism shown in FIG. 18 seen in a different direction.
FIG. 20A is a vertical sectional view showing first engagement members held in guide grooves according to a modification of the present invention.
FIG. 20B is a vertical sectional view showing first engagement members held in guide grooves according to a modification of the present invention.

### DESCRIPTION OF THE REFERENCE SYMBOLS

1, 50: endoscope apparatus, 2: insertion portion, 3: operation portion, 9: bending portion, 12: insertion-portion-side housing (first portion) 13, 52: main-unit-side housing (second portion), 14: operation lever, 16a, 16b, 16c, 16d, 26a, 26b, 26c, 26d: guide groove, 17: anchor groove, 19a, 19b, 19c, 19d: first engagement member, 20a, 20b, 20c, 20d: wire, 24a, 24b, 24c, 24d: coil spring, 27a, 27b, 27c, 27d, 57a, 57b, 57c, 57d: second engagement member, 31a, 31b, 31c, 31d: coil spring, 29a, 29b, 29c, 29d: operation wire, 34: guide pin, 45: lock lever, 45c: locking pawl, 47: release button, 48: cam, 53: remote controller, 54: arrow key, 59a, 59b, 59c, 59d: threaded screw, 81, 82: potentiometer, M1: first motor, M2: second motor

### BEST MODE FOR CARRYING OUT THE INVENTION

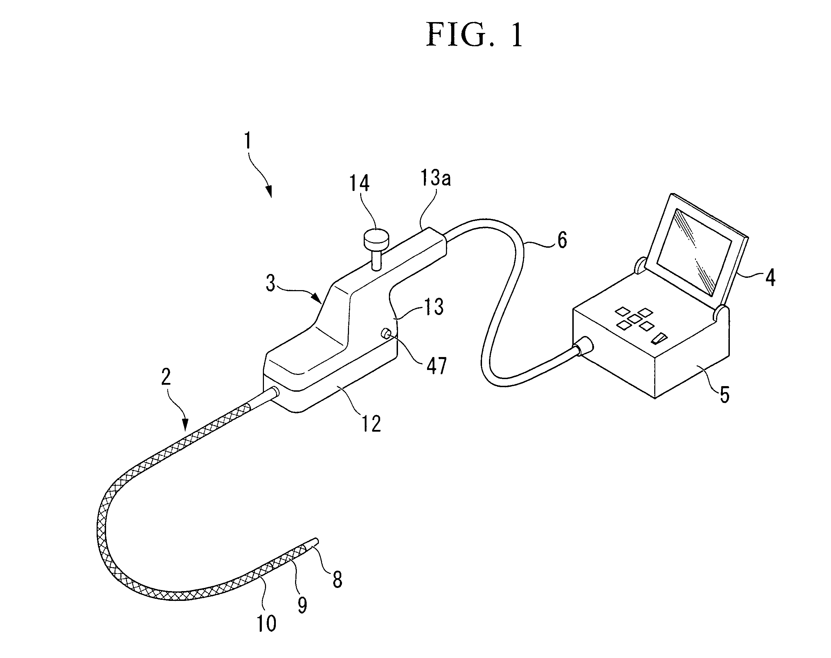

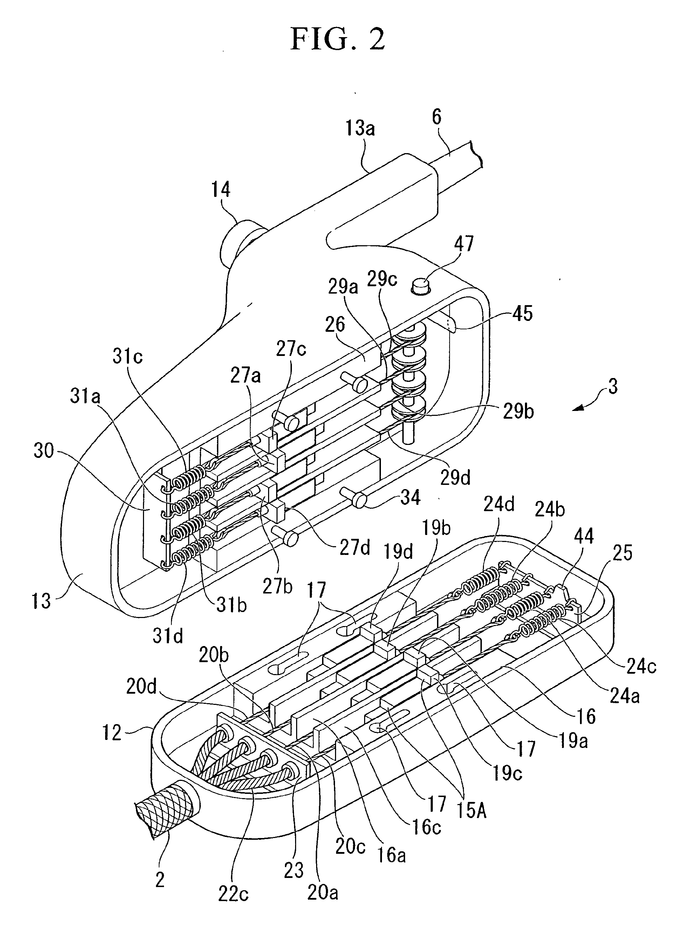

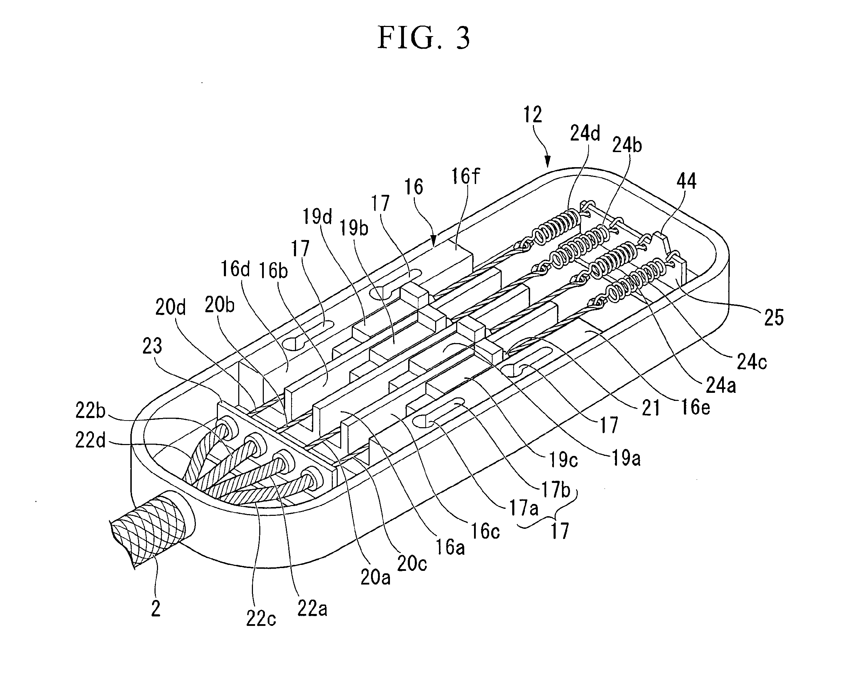

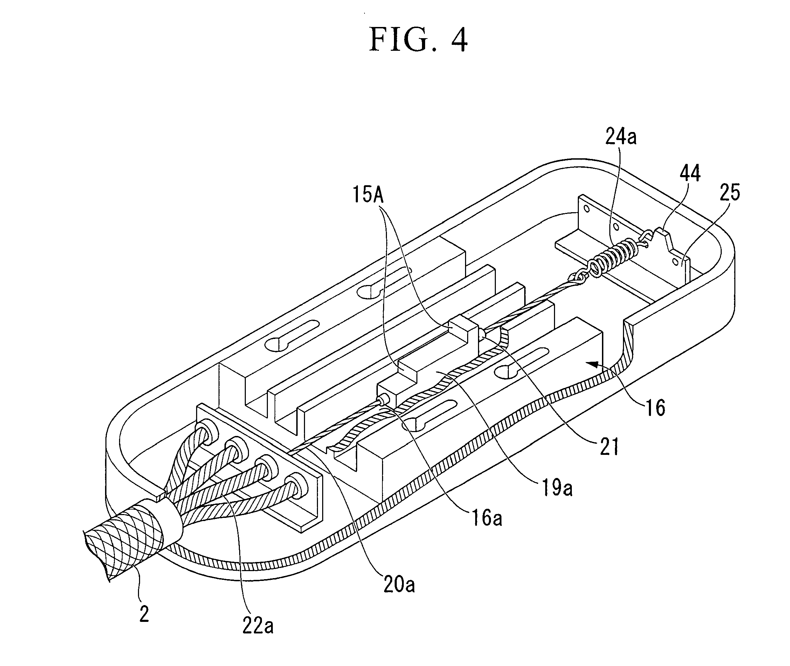

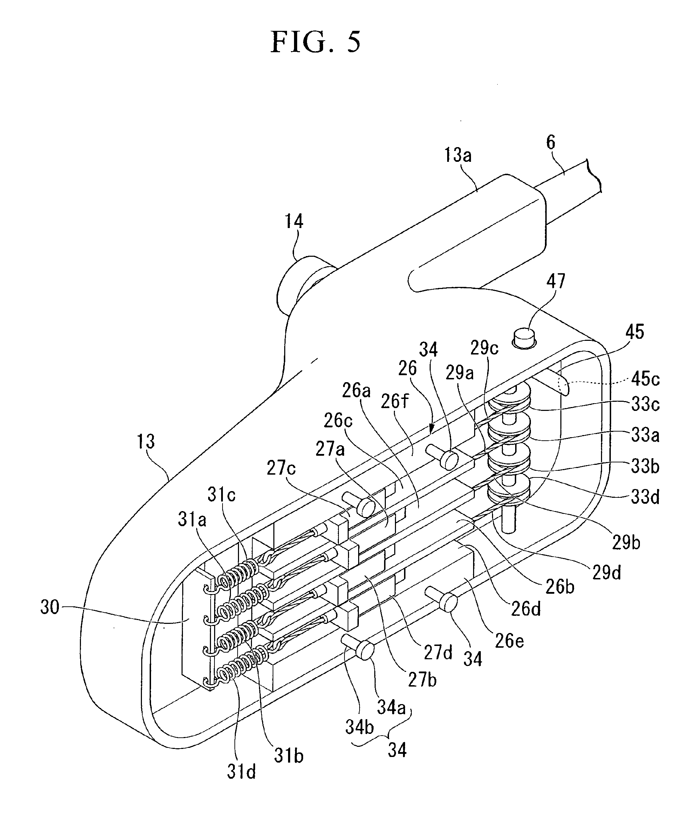

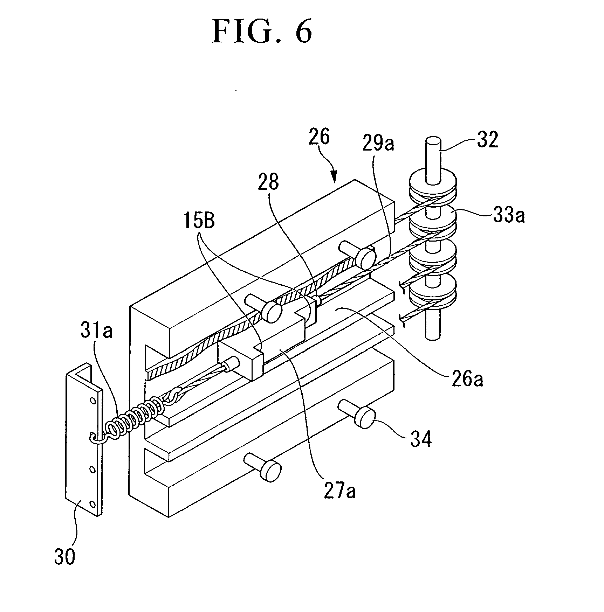

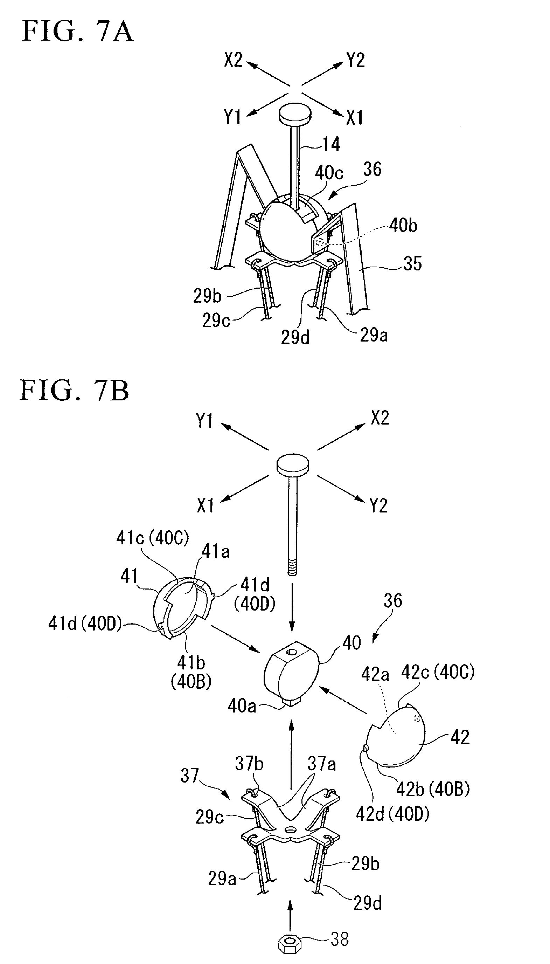

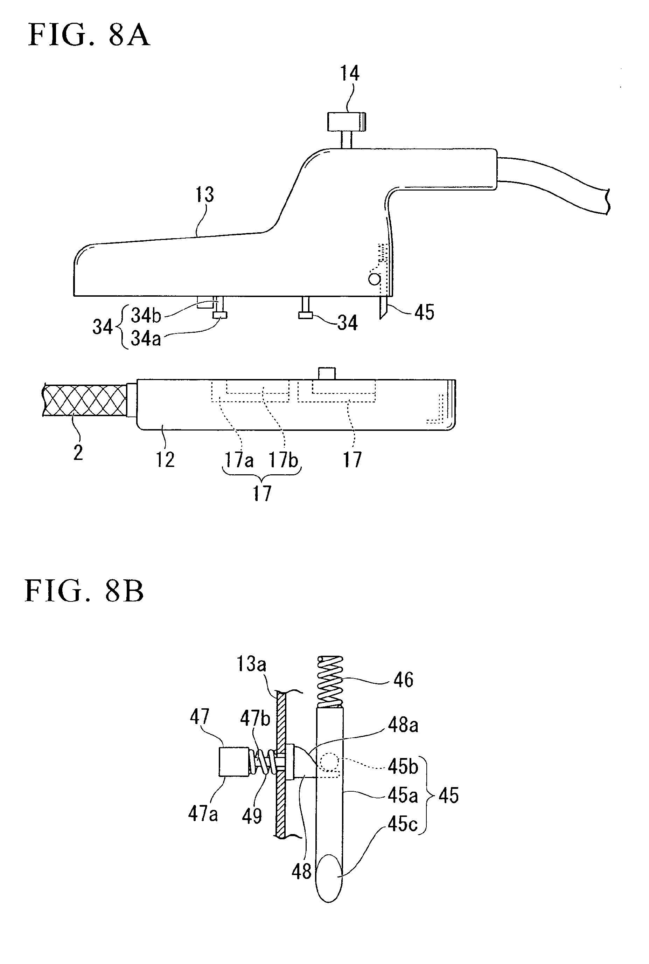

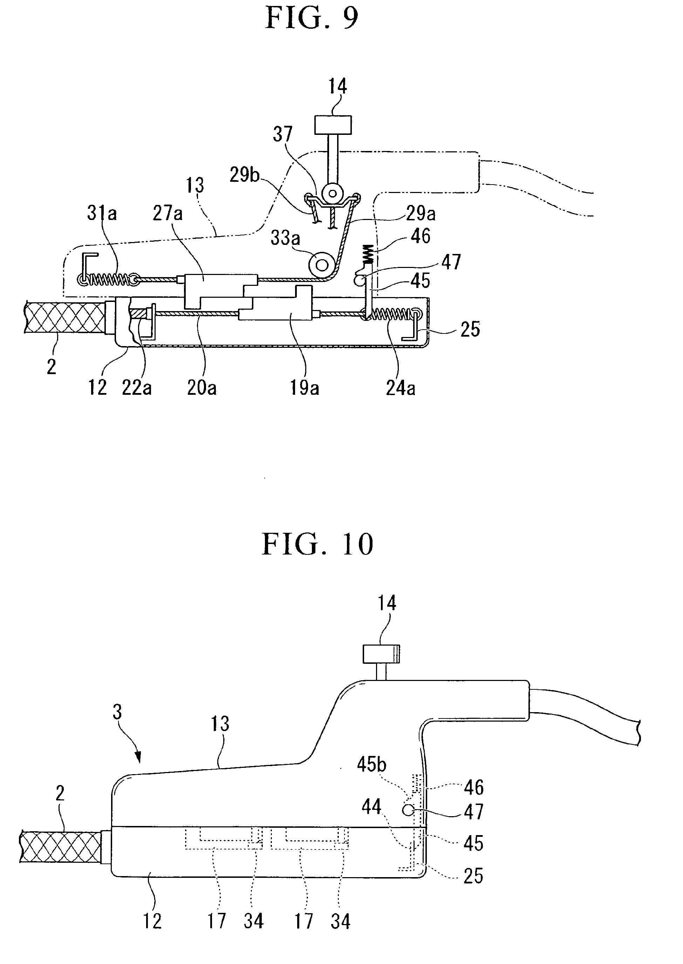

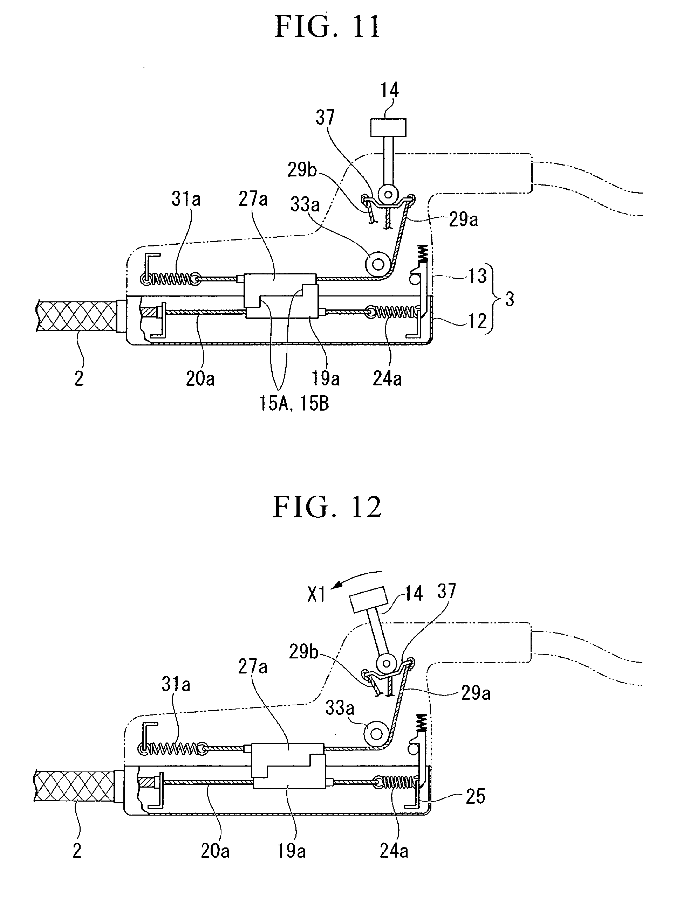

Hereinafter is a detailed description of embodiments of the present invention with reference to the drawings.
An endoscope apparatus according to a first embodiment of the present invention will be described with reference to FIG. 1 to FIG. 13. FIG. 1 is a diagram showing an entire construction of the endoscope apparatus according to the first embodiment of the present invention. FIG. 2 is an exploded perspective view showing an operation portion. FIG. 3 and FIG. 4 are diagrams showing a wire connection construction of an insertion-portion-side housing. FIG. 5 and FIG. 6 are diagrams showing a wire connection construction of a main-unit-side housing. FIG. 7A and FIG. 7B are a perspective view of the main part of an operation lever and an exploded view thereof, respectively. FIG. 8A and FIG. 8B are diagrams showing a lock construction of the insertion-portion-side housing and the main-unit-side housing. FIG. 9 and FIG. 10 are diagrams showing a construction of the operation portion before and after locking, respectively. FIG. 11 to FIG. 13 are diagrams showing operational states of the operation lever.
The endoscope apparatus 1 shown in FIG. 1 is roughly made of: a flexible, elongated insertion portion 2 that is to be inserted into a body cavity, a mechanical structure, or the like; an operation portion 3 that is provided at a proximal end of the insertion portion 2 for bending a bending portion 9 at a distal end of the insertion portion 2 upward and downward, and leftward and rightward; a controller 5 that has a power switch, an operation device, and the like for the endoscope apparatus 1 and is provided with a monitor 4; and a flexible universal cable 6 that connects the operation portion 3 with a controller 5.

In the endoscope apparatus 1, the insertion portion 2 is made of: a distal-end portion 8; a bending portion 9 that has bending pieces (not shown in the figure) provided consecutively thereinside and is capable of bending, for example, upward and downward, and leftward and rightward; and a flexible tube 10 that has bendability, in order from its distal end.
The operation portion 3 is coupled so as to be separable into an insertion-portion-side housing (first portion) 12 and a main-unit-side housing (second portion) 13 (see FIG. 2). In the main-unit-side housing 13, there is held an operation lever 14 that protrudes as an operation device from an upper opening thereof. In addition, there is formed a grip portion 13a that extends to the controller 5 side. The operation lever 14 that is used as a drive portion is capable of swaying in, for example, in the forward-backward and left-right directions. This makes it possible to bend the bending portion 9 of the insertion portion 2 via wires which will be described later.
In addition to the aforementioned construction, the endoscope apparatus 1 also includes the constructions of an observation system and an illumination system.
However, in the present embodiment, the description of those constructions is omitted.

Next is a description of an internal construction of the operation portion 3 based on FIG. 2 to FIG. 7.
In FIG. 2 to FIG. 4, in the insertion-portion-side housing 12 of the operation portion 3, there is arranged a guide plate 16 that has a plurality of guide grooves, for example four guide grooves 16a, 16b, 16c, 16d, which are formed in its central area of its longitudinal direction. In each of the frame portions 16e, 16f that sandwich the guide grooves 16a to 16d of the guide plate 16, there are formed, for example, two anchor grooves 17, 17 for coupling to the main-unit-side housing 13. When seen along the vertical section, the anchor groove 17 has a substantially rectangular shape in which a substantially L-shaped large-diameter groove portion 17a made of a vertical portion and a horizontal portion is in communication with a narrow-width groove 17b (see FIG. 8A).
In the guide grooves 16a, 16b, 16c, 16d of the guide plate 16, there are arranged first engagement members 19a, 19b, 19c, 19d slidably in the forward-backward direction, respectively. The first engagement members 19a to 19d has a substantially rectangular column-like shape with a two-step staircase-like step portion 15A formed on its upper surface as shown in, for example, FIG. 4. Through through-holes provided in the first engagement members 19a to 19d, there extend angle wires (hereinafter, referred to simply as wires) 20a, 20b, 20c, 20d as first pulling members, respectively. The wires 20a to 20d are fixed respectively on the first engagement members 19a to 19d with mouth rings 21 that are fixed on the proximal end side (controller 5 side) of the first engagement members 19a to 19d.

In FIG. 3, the proximal end of the insertion portion 2 is coupled to the insertion-portion-side housing 12. At this coupling portion, four angle coils 22a to 22d that extend from the interior of the insertion portion 2 to the interior of the housing 12 are coupled to a fixation plate 23 placed on the distal side of the guide plate 16, that is, placed on the insertion portion 2 side via the corresponding mouth ring. The wires 20a to 20d that respectively extend on the distal side of the first engagement members 19a to 19d extend into the interiors of the angle coils 22a to 22d, respectively. The wires 20a to 20d extend through the insertion portion 2, pass the top, bottom, left, and right sides of the bending pieces arranged in the bending portion 9, and then are fixed in the distal-end portion.
Here, in the wires 20a to 20d, for example the two wires 20a, 20b and the two wires 20c, 20d respectively form a set (pair). The wires 20a, 20b are fixed on the top and bottom sides of the distal-end portion (bending pieces) of the bending portion 9. The wires 20c, 20d are fixed on the left and right sides of the distal-end portion (bending pieces) of the bending portion 9. Therefore, when the wire 20a is pulled, the wire 20b is relaxed, and the bending portion 9 is bent in the up-down direction via the bending pieces. When the wire 20c is pulled, the wire 20d is relaxed, and the bending portion 9 is bent in the left-right direction.
Furthermore, on the base sides of the first engagement members 20a to 20d, there is fixed a plate-like spring retainer 25. On the spring retainer 25, there are hooked first ends of first biasing members, for example coil springs 24a to 24d. The second ends of the coil springs 24a to 24d are coupled respectively to the wires 20a to 20d that protruded from the mouth rings 21 of the first engagement members 19a to 19d. Therefore, the first engagement members 19a to 19d are held respectively in the guide grooves 16a to 16d in a manner slidable in the forward-backward direction while pulling the wires 20a to 20d with the biasing forces of the coil springs 24a to 24d.

Next is a description of the internal construction of the main-unit-side housing 13 based on FIG. 5 and FIG. 6. Similarly to the internal construction of the insertion-portion-side housing 12, the main-unit-side housing 13 includes: a guide plate 26 provided with guide grooves 26a to 26d; substantially rectangular column-like second engagement members 27a to 27d slidably held respectively in the guide grooves 26a to 26d; and operation wires 29a to 29d that extend respectively through the second engagement members 27a to 27d and function as actuation members or second pulling members. On each of the surfaces of the second engagement members 27a to 27d, there is formed a two-step staircase-like step portion 15B. Furthermore, the operation wires 29a to 29d are coupled respectively to the second engagement members 27a to 27d by means of a mouth ring 28 that is fixed on the distal end of each of the second engagement members 27a to 27d.
The second engagement members 27a to 27d are brought into engagement at the step portion 15B with the step portion 15A of the first engagement members 19a to 19d of the insertion-portion-side housing 12. It is possible for the step portions 15A and 15B to engage each other if each of them is provided with at least one step. However, a plurality of steps are preferably provided thereon because the torque produced in their engaged state can be reduced.
On the distal side of the main-unit-side housing 13, that is, on the insertion portion 2 side, there is fixed a plate-like spring retainer 30. On the spring retainer 30, there are hooked first ends of second biasing members, for example coil springs 31a to 31d. The second ends thereof are coupled to the operation wires 29a to 29d, respectively. Thereby, the second engagement members 27a to 27d are held respectively in the guide grooves 26a to 26d in a manner slidable in the forward-backward direction while pulling the operation wires 29a to 29d with the biasing forces of the coil springs 31a to 31d.
Furthermore, on the proximal end side of the main-unit-side housing 13, pulleys 33a to 33d are rotatably supported by a pulley shaft 32. The direction of the operation wires 29a to 29d is changed toward the operation lever 14, and are wound around the pulleys 33a to 33d, respectively.

Next is a description of an actuation mechanism of the operation wires 29a to 29d by the operation lever 14 based on FIG. 7A and FIG. 7B.
In FIG. 7A and FIG. 7B, the operation lever 14 extend into the interior of the main-unit-side housing 13 through its opening, and is capable of swaying in the X1-X2 direction and in the Y1-Y2 direction by a bearing 36 that is supported by frames 35. The X1-X2 direction and the Y 1-Y2 direction are orthogonal to each other so as to form a substantially cross shape. To the bottom portion of the bearing 36, there is coupled a substantially cross-shaped arm portion 37 via a fixation portion 40a.
As shown in FIG. 7B, the bearing 36 is mainly made of: a round shaft 40 with a fixation portion 40a; and a pair of spherical bearings 41, 42. The round shaft 40 is formed into a substantially cylindrical shape. The operation lever 14 extends through the round shaft 40 and the arm portion 37. With a nut 38 screwed around a male thread portion at the bottom of the operation lever 14, the operation lever 14 fixes the round shaft 41 and the arm portion 37 coaxially.

The first spherical bearing 41 and the second spherical bearing 42 are formed into a hemispherical shape. In the interiors of the first spherical bearing 41 and the second spherical bearing 42, there are respectively formed round bearing portions 41 a, 42a into which the round shaft 40 is to be fit. In the first spherical bearing 41 and the second spherical bearing 42, there are respectively provided: first notch portions 41 b, 42b that constitute a groove portion 40B in which the fixation portion 40a provided on the round shaft 40 is arranged; and second notch portions 41 c, 42c that constitute a groove portion 40C in which the operation lever 14 fixed on the round shaft 41 is arranged. Furthermore, in the first spherical bearing 41 and the second spherical bearing 42, there are respectively provided a pair of semi-cylindrical portions 41 d, 42d at opposite positions. When integrated, the semi-cylindrical portions 41d, 42d constitute cylindrical shaft portions 40D.
In the arm portion 37, there are protrudingly formed four arm portions 37a that respectively extend in the X1, X2 directions and the Y1, Y2 directions at substantially every 90° in the circumferential direction. In each of the arm portions 37a, a wire fixation hole 37b is bored. The operation wires 29a to 29d that have been wound respectively around the pulleys 33a to 33d and are directed toward the operation lever 14 are fixed through the wire fixation holes 37b of the arm portions 37a, respectively.
The bearing 36 is made of the round shaft 40, to which the arm member 37 and the operation lever 14 are coaxially attached and fixed, being sandwiched between the first spherical bearing 41 and the second spherical bearing 42. Therefore, the round shaft 40 is capable of swaying in the arrow X1 direction and the arrow X2 direction within the range of groove portions 40B, 40C.
Furthermore, with the round shaft 40 being sandwiched between the first spherical bearing 41 and the second spherical bearing 42, the semi-cylindrical portions 41d, 42d are integrated into the coaxial shaft portions 40D, 40D. The shaft portions 40D, 40D are rotatably supported as rotation shafts at predetermined positions of the frame 35. Therefore, the bearing 36 is capable of swaying in the arrow Y1 direction and the arrow Y2 direction.

Consequently, when the operation lever 14 is put in an erect state as shown in FIG. 7A, the portions of the operation wires 29a to 29d from the pulleys 33a to 33d toward the arm member 37 are all in a predetermined relaxed state. In this state, the operation lever 14 is operated in the arrow X1 direction or the arrow X2 direction, or in the arrow Y1 direction or the arrow Y2 direction in the figures. Thereby, with the inclining operation of the operation lever 14, the arm member 37 sways.
When the tension of any of the operation wires 29a to 29d attached to the fixation holes 37b of the arm portions 37a that corresponds to the direction of inclination of the operation lever 14, for example, the tension of the operation wire 29a is put in a pulled state from a relaxed state, then the resistance of the operation wire 29a to the pulley increases, which in turn increases the resistance between the operation wire 29a and the pulley 33a around which the operation wire 29a is wound. On the other hand, the opposed operation wire 29b is put in a further relaxed state.

Next is a description of a connection construction between the insertion-portion-side housing 12 and the main-unit-side housing 13.
In the guide plate 26 of the main-unit-side housing 13 shown in FIG. 5, (for example, two) guide pins 34, 34 that the engage anchor grooves 17, 17 of the insertion-portion-side housing 12 are provided on each of the frame portions 26e, 26f that sandwich the guide grooves 26a to 26d. The guide pins 34 are used as guide members. As shown in FIG. 8A, the guide pin 34 is made of a large-diameter head portion 34a and a shaft portion 34b. The head portion 34a of the guide pin 34 is configured to have a size that is insertable into a large-diameter groove portion 17a of the anchor groove 17 but is not insertable into a narrow-width groove 17b thereof.
Therefore, to connect the insertion-portion-side housing 12 with the main-unit-side housing 13, the housings 12, 13 are opposed in a state with their opening portions are displaced, and the head portions 34a of the guide pins 34 are inserted into the vertical portions of the large-diameter groove portions 17a of the anchor grooves 17. In this state, the insertion-portion-side housing 12 and the main-unit-side housing 13 are displaced from each other in the direction in which the step portion 15A and the step portion 15B are further away from each other. Next, either one of the insertion-portion-side housing 12 and the main-unit-side housing 13 is slid. This fits the head portions 34a of the guide pins 34 into the horizontal portions of the substantially L-shaped large-diameter groove portions 17a of the anchor grooves 17. Thereby, the housings 12, 13 are mutually aligned into a connected state.

Next is a description of a positioning-fixation mechanism (lock construction) for the insertion-portion-side housing 12 and the main-unit-side housing 13 based on FIG. 8A and FIG. 8B.
In the main-unit-side housing 13 shown in FIG. 8A, there is provided a lock lever 45 as a lock member to the rear of the pulleys 33a to 33d. In FIG. 8B, the lock lever 45 is made of a long-shafted lever portion 45a and a camshaft 45b that protrudes in the direction substantially orthogonal thereto. The lever portion 45a is biased downward by a push spring 46which is provided at the top thereof in a compressed state. The bottom edge surface of the lever portion 45a is a locking pawl 45c with an inclined surface.
Through a side wall 13a of the main-unit-side housing 13, there is mounted a release button 47 as a release member. The release button 47 has a release cam 48 provided at its distal end. An inclined-surface-like cam surface 48a of the release cam 48 locks the camshaft 45b of the lock lever 45. A head portion 47a of the release button 47 protrudes to the outside of the side wall 13a. A return spring 49 is placed around a shaft portion 47b between the head portion 47a and the side wall 13a. Pressing the head portion 47a of the release button 47 causes the cam surface 48a to retract (lift) the lock lever 45. Pulling the head portion 47a allows the release cam 48 to retract from the camshaft 45b with an elastic force of the return spring 49.

On the other hand, in the insertion-portion-side housing 12, there is protrudingly provided a stopper 44 on a part of the spring retainer 25. When the main-unit-side housing 13 is connected with the insertion-portion-side housing 12, the lock lever 45 typically protrudes lower than the opening portion of the main-unit-side housing 13.
When the main-unit-side housing 13 is slid with respect to the insertion-portion-side housing 12 in a state with the guide pins 34 inserted into the anchor grooves 17, the locking pawl 45c of the lock lever 45 abuts the stopper 44, retracts while resisting the biasing force of the push spring 46. Subsequently, the locking pawl 45c moves over the stopper 44 and again moves down. Then, in a state with the head portions 34a of the guide pins 34 having reached the farthest sections of the horizontal portions in the large-diameter groove portions 17a of the anchor grooves 17, the housings 12, 13 are mutually aligned, the lock lever 45 is locked between the stopper 44 and the proximal end side wall of the insertion-portion-side housing 12, to thereby put the housings 12, 13 in a locked state in which misalignment between them is prevented.
To release the locked state requires only that the main-unit-side housing 13 be displaced from the insertion-portion-side housing 12 in the direction of releasing the fitting between the head portions 34a of the guide pins 34 and the horizontal portions in the large-diameter groove portions 17a of the anchor grooves 17, in a state where the lock lever 45 being retracted from the stopper 44 by pressing the release button 47.

The operation apparatus of the endoscope apparatus 1 according to the present embodiment has the aforementioned construction. Next is a description of its operation.
When the endoscope apparatus 1 as shown in FIG. 1 is used to perform an observation, an image-pickup, a treatment, or the like of the interior of a body cavity or a mechanical structure, the operation lever 14 is inclined in any direction of X1, X2, Y1, and Y2. Thus, the bending portion 9 of the operation portion 2 is bent in any of the up, down, left, and right directions. Thereby, it is possible to perform an observation with the bending portion 9 directed to an optional direction. When the insertion portion 2 is replaced with one of another type, the release button 47 is pressed in the state shown in FIG. 10 to retract the lock lever 45 and release the engagement with the stopper 44.
In this state, the insertion-portion-side housing 12 and the main-unit-side housing 13 are relatively slid in the opposite directions, to thereby move the guide pins 34 of the main-unit-side housing 13 along the horizontal portions in the large-diameter groove portions 17a of the anchor grooves 17 of the insertion-portion-side housing 12 (see FIG 9). At the time when the head portions 34a of the guide pins 34 has reached the end portions of the substantially L-shaped large-diameter groove portions 17a after the housings 12, 13 are displaced in the horizontal direction, the housings 12, 13 are pulled apart from each other in the spacing direction. Thereby, the guide pins 34 are moved along the vertical portions in the large-diameter groove portions 17 of the anchor groove 17, allowing for the separation of the housings 12, 13 (see FIG. 8A).

Next, another insertion-portion-side housing 12 is connected with the main-unit-side housing 13 in the steps reverse to the aforementioned separation steps. To be more specific, the guide pins 34 of the main-unit-side housings 13 are inserted into the vertical portions in the substantially L-shaped large-diameter groove portions 17a of the anchor grooves 17 of the insertion-portion-side housings 12 (see FIG. 8A). At this time, in a state with the opening portions of the housings 12, 13 being in abutment with each other in a displaced manner, the step portions 15B, 15A are held in a spaced manner while the first engagement members 19a to 19d of the insertion-portion-side housing 12 are opposed to the second engagement members 27a to 27d in the guide grooves 16a to 16d, 26a to 26d.
Then, the main-unit-side housing 13 is relatively slid with respect to the insertion-portion-side housing 12. In this slide operation, the head portions 34a of the guide pins 34 are guided while being moved along the horizontal portions in the large-diameter groove portions 17a of the anchor grooves 17 of the insertion-portion-side housings 12 in their extension direction. This brings the second engagement members 27a to 27d into abutment with the first engagement members 19a to 19d at the step portions 15B, 15A. At this time, the first and second engagement members 19a to 19d, 27a to 27d are slidably held in the guide grooves 16a to 16d, 26a to 26d. Therefore, the engaged step portions 15A, 15B will never be displaced.

Consequently, for example if, of the operation wires 29a and 29b or the operation wires 29c and 29d that are anchored by the opposing arm portions 37a, 37a of the arm members 37, one is in a relaxed state and the other is in a pulled state, then some or all of the second engagement members 27a to 27d are at positions displaced forward and backward in the sliding direction. Hence, the operation lever 14 is positioned at an inclined position in accordance with the displacements.
Even in this case, with the first engagement members 19a to 19d and the second engagement members 27a to 27d being in abutment with each other, unevenness in relaxation and tension among the operation wires 29a to 29d is removed. Accordingly, in connecting the housings 12, 13, it is not necessary to adjust the positions of the first engagement members 19a to 19d and the second engagement members 27a to 27d in advance.
This similarly applies to the insertion portion 2 and the bending portion 9. That is, even if the bending portion 9 is bent in any of the up, down, left, and right directions when the insertion-portion-side housing 12 is connected with the main-unit-side housing 13, the guide pins 34 of the main-unit-side housings 13 are inserted into the anchor grooves 17 of the insertion-portion-side housings 12, and then the first and second engagement members 19a to 19d, 27a to 27d are slid from a spaced state into their mutual abutment. Thereby, unevenness in relaxation and tension among the wires 20a to 20d is removed.

Next is a description of an operation method of the endoscope apparatus 1 using the operation portion 3 in which the insertion-portion-side housing 12 and the main-unit-side housing 13 are coupled, based on FIG. 11 to FIG. 13.
As described above, in FIG. 11, the first and second engagement members 19a to 19d, 27a to 27d engage each other in a state with the insertion-portion-side housing 12 and the main-unit-side housing 13 of the operation portion 3 being coupled. At this time, the operation lever 14 and the insertion portion 2 are in their neutral positions. From this state, if the operation lever 14 is inclined in, for example, the X1 direction as shown in FIG. 12, the operation wire 29a is pulled by the inclined arm member 37. This slides the second engagement member 27a backward along the guide groove 26a while resisting the biasing force of the coil spring 31a. Therefore, the first engagement member 19a in engagement at the step portions 15B are pushed and slid backward along the guide groove 16a. As a result, the coil spring 24a is compressed and the wire 20a is pulled.

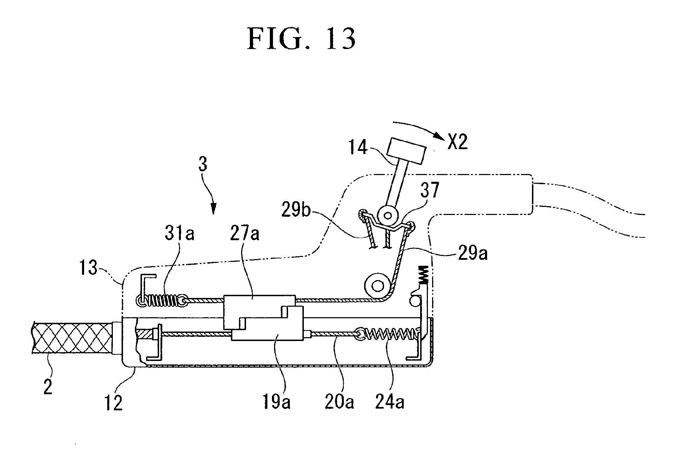

Furthermore, with the inclination of the operation lever 14 and the arm member 37, the operation wire 29b opposite to the operation wire 29a is relaxed. Then, the second engagement member 27b is pulled by the biasing force of the coil spring 31 b to be moved forward. Accordingly, the second engagement member 27b is spaced from first engagement member 19b to relax the tension applied to the wire 20b. Therefore, with the inclination of the operation lever 14 in the X1 direction as described above, the wire 20a is pulled, which imparts, for example, a downward bending force to the bending portion 9 at the distal end of the insertion portion 2. In addition, the wire 20b is relaxed, which bends the bending portion 9 downward.
Furthermore, as shown in FIG. 13, if the operation lever 14 is inclined in the X2 direction, the operation wire 29b is pulled and the operation wire 29a is relaxed, which is contrary to the above case. Thereby, the second engagement member 27a is spaced from the first engagement member 19a to relax the wire 20a. At the same time, the second engagement member 27b pushes and moves the first engagement member 19b while resisting the biasing force of the coil spring 31, and the wire 20b is pulled. As a result, the bending portion 9 at the distal end of the insertion portion 2 is bent upward because the wire 20b is pulled and also the wire 20a is relaxed. At this time, the wire 20a may move with a time lag, which may cause a slack in the wire 20a. However, the second engagement member 27a and the first engagement member 19a are not fixed but separable. Therefore, the wire 20a is not forcefully moved forward, which does not lead to an undesirable situation where the wire 20a becomes slack.

Thus, with the inclination of the operation lever 14 in the X1 direction or the X2 direction, the bending portion 9 at the distal end of the insertion portion 2 can be bent downward or upward. Similarly, with the inclination of the operation lever 14 in the Y1 direction or the Y2 direction, the bending portion 9 at the distal end of the insertion portion 2 can be bent leftward or rightward.

As described above, according to the operation apparatus of the endoscope apparatus 1 according to the present embodiment, only an attachment of an integrally-coupled unit of an insertion portion 2 and an insertion-portion-side housing 12 to the main-unit-side housing 13 completes a replacement with one of another type, and enables an observation, an image-pickup, a treatment, or the like.
Furthermore, in setting to the neutral position, the guide pins 34 of the main-unit-side housing 13 are inserted into the vertical portions in the large-diameter groove portions 17a of the anchor grooves 17 of the insertion-portion-side housing 12, and the two are shifted from a state where they are opposed to each other at mutually displaced positions to a coupled state where the guide pins 34 are slid to the farthest sections of the horizontal portions in the large-diameter groove portions 17a. With such simple operations, the first engagement members 19a to 19d and the second engagement members 27a to 27d, which are opposed to each other in a spaced manner, can be engaged with each other and positionally adjusted to their respective neutral positions.
Therefore, even if before coupling, the operation lever 14 is inclined in any direction, or the bending portion of the insertion portion 2 is bent in any direction, only coupling the insertion-portion-side housing 12 to the main-unit-side housing 13 makes it possible to adjust the displacement between the first engagement members 19a to 19d and the second engagement members 27a to 27d into their respective neutral states. In addition, in replacing the insertion portion 2 and the insertion-portion-side housing 12, it is not necessary to previously position or align the first and second engagement members 19a to 19d, 27a to 27d, which connect the operation lever 14 with the wires 20a to 20d of the insertion portion 2, to their neutral positions, specific positions, or the like. Only impartment of a required pressure with the first engagement members 19a to 19d being in abutment with the second engagement members 27a to 27d makes it possible to adjust the displacement between the engagement members 19a to 19d, 27a to 27d into their neutral states.
Accordingly, only sliding and coupling the insertion-portion-side housing 12 and the main-unit-side housing 13 makes it possible to set the operation lever 14 and the bending portion 9 of the insertion portion 2 to their neutral positions.

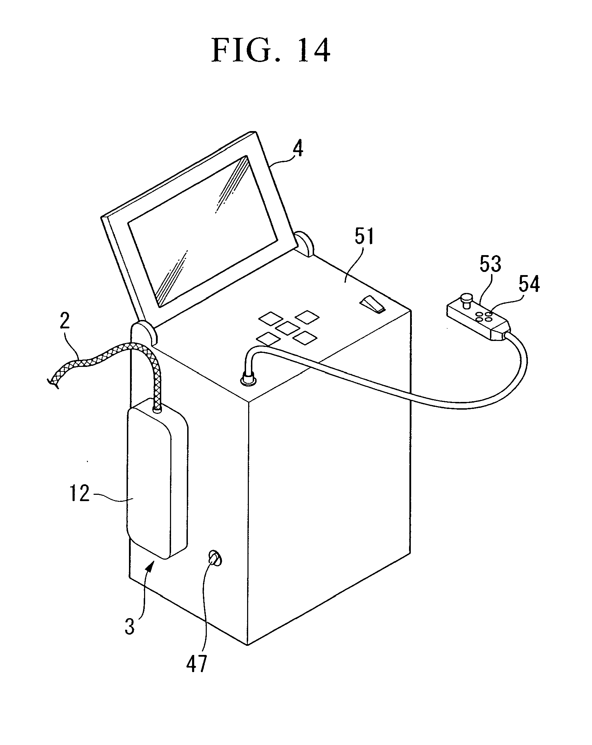

The present invention is not limited to the aforementioned first embodiment. Appropriate modifications can be made without departing from the spirit or scope of the present invention.
Next is a description of an operation apparatus of an endoscope apparatus 50 according to a second embodiment of the present invention based on FIG. 14 to FIG. 19. Portions or members the same as or similar to those of the operation apparatus of the endoscope apparatus 1 according to the aforementioned embodiment are denoted by the same reference symbols, and description thereof is omitted.

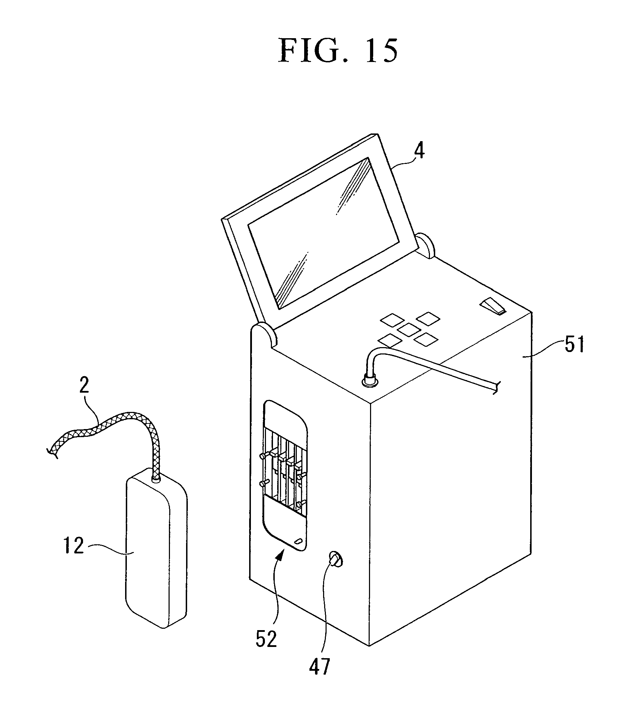

FIG. 14 and FIG. 15 are perspective views of the main part of an electrically-bending type endoscope apparatus 50. In the electrically-bending type endoscope apparatus 50 according to the present embodiment, it is configured such that a bending portion 9 of an insertion portion 2 is bent in the up, down, left, and right directions with electromotion by motor drive. In the figures, the insertion portion 2 and an insertion-portion-side housing 12 are identical in construction to those of the first embodiment.
In a controller 51 provided with a monitor 4, there is embedded a main-unit-side housing 52 for coupling-connection with the insertion-portion-side housing 12. That is, in the present embodiment, the controller 51 and the main-unit-side housing 52 are integrally constructed. Furthermore, in the vicinity of the main-unit-side housing 52, there is provided a release button 47 for detaching the insertion-portion-side housing 12. The positioning-fixation mechanism (lock construction) including the release button 47 for the insertion-portion-side housing 12 and the main-unit-side housing 52 is the same as that of the aforementioned first embodiment, although it lies outside an operation portion 3. To the controller 51, there is provided a remote controller 53 as an operation device via a cord. To a remote controller 53, there is provided, for example as an arrow key 54, an operation button for bending the bending portion 9 of the insertion portion 2 in the up-down direction and the left-right direction. Pressing any of the four edge portions of the arrow key 54 makes it possible to bendingly operate the bending portion 9 in the up, down, left, and right directions.

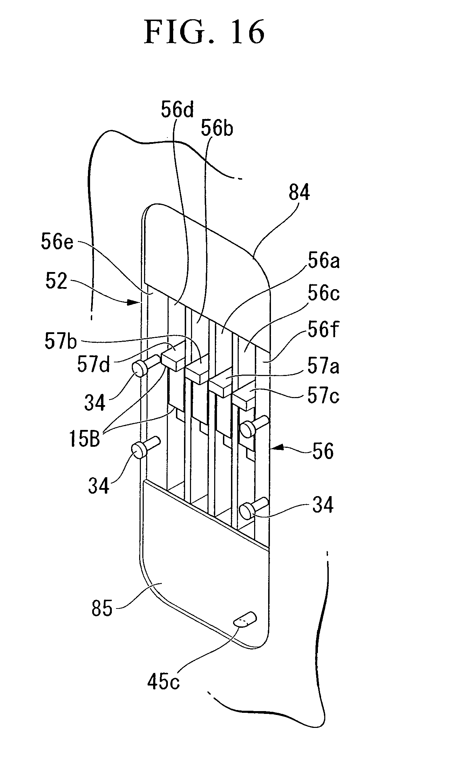

The construction of the main-unit-side housing 52 will be described with reference to FIG. 16 to FIG. 19.
In the main-unit-side housing 52 shown in FIG. 16, similarly to the internal construction of the insertion-portion-side housing 12, there are provided: a guide plate 56 including guide grooves 56a to 56d in the middle portion in its longitudinal direction (in the up-down direction, in the figure); and substantially rectangular column-like second engagement members 57a to 57d that are slidably held respectively in the guide grooves 56a to 56d. Each of the second engagement members 57a to 57d has a staircase-like step portion 15B with, for example two steps, on its surface. It is configured such that the second engagement members 57a to 57d are slidable respectively in the guide grooves 56a to 56d in the up-down direction.
The second engagement members 57a to 57d are to respectively engage the first engagement members 19a to 19d of the insertion-portion-side housing 12 at the step portions 15B, 15A. It is possible for the step portions 15A and 15B to engage each other if each of these step portions has at least one step. However, a plurality of steps are preferably provided on the two because the torque produced between the engaged surfaces can be reduced.

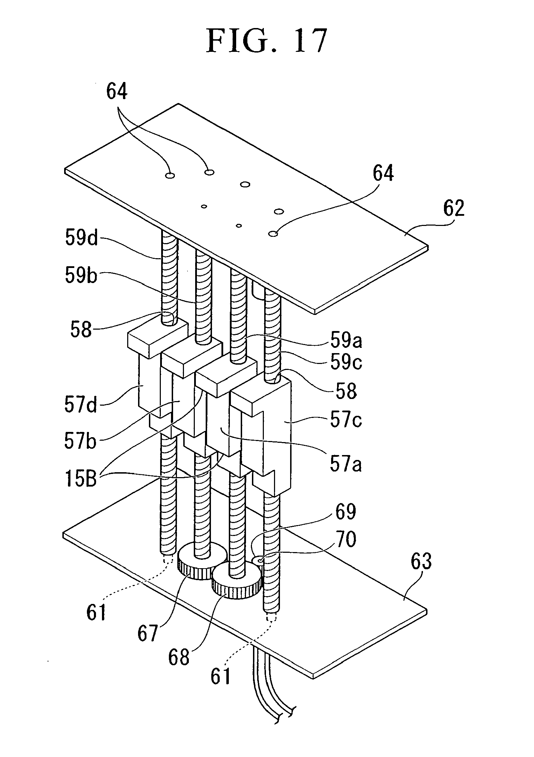

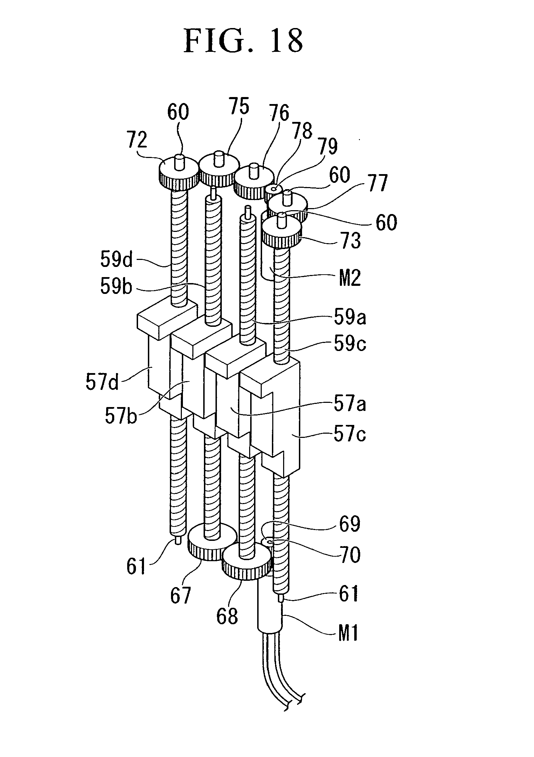

As shown in FIG. 17, in each interior of the second engagement members 57a to 57d, there is formed an internally threaded screw holes 58 through each of the second engagement members 57a to 57d in its longitudinal direction. Extend screw members as direct drive mechanisms, for example threaded screws 59a to 59d, are inserted into the screw holes 58 of the second engagement members 57a to 57d in the up-down direction in a screwed manner. The direct drive mechanisms such as the threaded screws 59a to 59d function as actuation members. On the upper and lower end portions of each of the threaded screws 59a to 59d that are screwed respectively through the second engagement members 57a to 57d, there are formed an upper shaft 60 and a lower shaft 61, respectively (see FIG. 18). As shown in FIG. 17, for example the four upper shafts 60 and the four lower shafts 61 are rotatably supported respectively in through-holes 64, 65 that are provided respectively in an upper bearing plate 62 an a lower bearing plate 63.
Note that, instead of the upper bearing plate 62 with the through-holes 64 and the lower bearing plate 63 with the through-hole 65, there may be provided bearings for every one of the threaded screws 59a to 59d.

As shown in FIG. 17 to FIG. 19, onto the lower end portions of the threaded screws 59a, 59b, a first flat gear 67 and a second flat gear 68 are fixed above the lower shafts 61, respectively. The first flat gear 67 and the second flat gear 68 are in mesh with each other. One flat gear, for example the second flat gear 68 is in mesh with a pinion gear 69. Into the pinion gear 69, there is coaxially secured a motor shaft 70 of a first motor M1 for up-down movement. Note that the gears 67, 68, 69 are referred to as a first interlock mechanism.
The first motor M1 is capable of rotating in the normal and reverse directions. The first motor M1 transmits a rotational force to the first and second flat gears 67, 68 so that for example its rotation in the normal direction moves the second engagement member 57a upward and the second engagement member 57b downward, and so that its rotation in the reverse direction moves the second engagement member 57a downward and the second engagement member 57b upward.
Furthermore, onto the upper end portions of the threaded screws 59c, 59d, a third flat gear 72 and a fourth flat gear 73 are fixed below the upper shafts 60, respectively.
For example, with the third flat gear 72, there are held a first interlocking gear 75 and a second interlocking gear 76 in a sequentially meshed state. With the fourth flat gear 73, there is held a third interlocking gear 77 in a meshed manner. Between the second interlocking gear 76 and the third interlocking gear 77, there is held a pinion gear 78 in a meshed state. Into the pinion gear 78, there is coaxially fixed a motor shaft 79 of a second motor M2 for left-right movement. Note that the gears 76, 75, 72, 77, 73, 78 are referred to as a second interlock mechanism.
The second motor M2 is also capable of rotating in the normal and reverse directions. The second motor M2 transmits a rotational force sequentially to the gears 76, 75, 72, 77, 73 so that for example its rotation in the normal direction moves the second engagement member 57c leftward and the second engagement member 57d rightward, and so that its rotation in the reverse direction moves the second engagement member 57c rightward and the second engagement member 57d leftward.

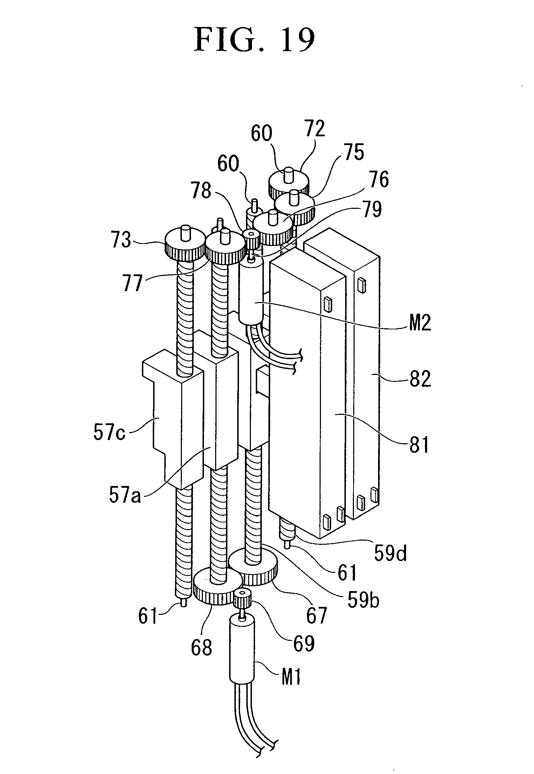

Furthermore, as shown in FIG. 19, the first motor M1 is electrically connected to an up-down-direction potentiometer 81. In the potentiometer 81, for example a neutral position of the first motor M1 is previously stored. Furthermore, movement positions of the second engagement members 57a, 57b from the neutral position by the normal/reverse rotation of the first motor M1, that is, upward/downward bend angles of the bending portion 9 from the neutral position may be previously measured and stored therein.
Similarly, the second motor M2 is electrically connected to a left-right-direction potentiometer 82. In the potentiometer 82, for example a neutral position of the second motor M2 is previously stored. Furthermore, movement positions of the second engagement members 57c, 57d from the neutral position by the normal/reverse rotation of the second motor M2, that is, leftward/rightward bend angles of the bending portion 9 from the neutral position may be previously measured and stored therein
The first and second motors M1, M2, the potentiometers 81, 82, and the first and second interlock mechanisms function as a drive portion. The first and second motors M1, M2 function as a drive portion or a drive source. The first and second motors M1, M2 need not necessarily be provided in the main-unit-side housing 52. They may be provided in the controller 51.
Furthermore, referring to FIG. 16, on both sides of the group of the guide grooves 56a to 56d in the guide plate 56 in the main-unit-side housing 52, there are respectively provided frame portions 56e, 56f, and there are respectively implanted therein a pair of guide pins 34, 34. The guide pins 34 can be fitted into anchor grooves 17 of the insertion-portion-side housing 12 and is slidable therealong; which is similar to the case of the first embodiment.
Furthermore, in the main-unit-side housing 52, an upper cover 84 and a lower cover 85 are provided on top and bottom of the guide plate 56, respectively. From an opening of the lower cover 85, there is protruded a locking pawl 45c of a lock lever 45.

The operation apparatus of the electrically-bending type endoscope apparatus 50 according to the present second embodiment includes the aforementioned construction. Next is a description of its operation.
First, when the insertion portion 2 including the insertion-portion-side housing 12 is to be replaced with another type of insertion portion 2, pressing a release button 47 of a controller 51 retracts the locking pawl 45c of the lock lever 45 to release the engagement of the insertion-portion-side housing 12 with a stopper 44. When the insertion-portion-side housing 12 is slid upward at the same time, the anchor grooves 17 of the insertion-portion-side housing 12 is slid with respect to the guide pins 34 of the main-unit-side housing 52, to thereby make it possible to remove the insertion-portion-side housing 12 from the main-unit-side housing 52.
Next, with regard to a main-unit-side housing 12 provided with an insertion portion 2 to be attached, its anchor grooves 17 are attached onto the guide pins 34 of the main-unit-side housing 52, and then are slid along the main-unit-side housing 52.
Thereby, the locking pawl 45c of the lock lever 45 moves over the stopper 44. Thus, the stopper 44 together with the anchor grooves 17 and the guide pins 34 can attachedly fix the insertion-portion-side housing 12 into a locked state.

In a state where the new insertion-portion-side housing 12 is attached to the main-unit-side housing 52, the second engagement members 57a to 57d held respectively at appropriate positions in the guide grooves 56a to 56d of the main-unit-side housing 52 attached to the controller 51 are screwed to the threaded screws 59a to 59d. Therefore, the first engagement members 19a to 19d of the insertion-portion-side housing 12, when brought into abutment respectively with the second engagement members 57a to 57d, are forcefully positioned. The positions of the first engagement member 19a to 19d in this case are adjusted respectively by the biasing forces of the coil springs 24a to 24d.
At this time, even if there is a slack and a tension in a set or two of the wires of the first engagement members 19a to 19d for the left-right direction or up-down direction because the bending portion 9 is bent in any direction and the first engagement members 19a to 19d are not in their neutral positions, their positions are adjusted by the first engagement members 19a to 19d being brought into abutment with any of the second engagement members 57a to 57d.
Subsequently, the arrow key 54 is operated to drive the first motor M1 and the second motor M2. This moves the second engagement members 57a to 57d to their respective neutral positions stored in the potentiometers 81, 82. Then, the first engagement members 19a to 19d are followingly moved to their respective neutral positions while their tensions are being adjusted by the biasing forces of the coil springs 24a to 24d.

Next, in the case where the endoscope apparatus 50 shown in FIG. 14 is used to perform an observation or the like of the interiors of a body cavity and a mechanical structure, one of the edge portions of the arrow key 54 of the remote controller 53 is pressed to drive the first motor M1 for up-down movement or the second motor M2 for left-right movement. Thereby, the bending portion 9 of the insertion portion 2 is bent.
For example, when the arrow key 54 is used to rotate the second motor M2 in the normal direction, the rotation of the pinion gear 78 is transmitted to the third flat gear 72 via the second and first interlocking gears 76, 75. This rotates threaded screw 59d integrally with the third flat gear 72 in one direction to move the second engagement member 57d upward. At the same time, the rotation is transmitted to the fourth flat gear 73 via the third interlocking gear 77. This rotates the threaded screw 59c in the reverse direction synchronously with the fourth flat gear 72, thus moving the second engagement member 57c downward.
Then, in conjunction with the up and down movements of the second engagement members 57c, 57d, the first engagement members 19d, 19c of the insertion-portion-side housing 12 move up and down, respectively. Therefore, the wire 20d is relaxed and the wire 20c is pulled, which bends the bending portion 9 of the insertion portion 2 leftward or rightward. Also in the case where another edge portion of the arrow key 54 is pressed, the bending portion 9 of the insertion portion 2 is bent in an appropriate direction by a similar operation.

Also in the endoscope apparatus 50 according to the second embodiment, the insertion-portion-side housing 12 is attached to the main-unit-side housing 52 that is attached to the controller 51. Then, the first engagement members 19a to 19d of the insertion-portion-side housing 12 are slid with respect to the second engagement members 57a to 57d that are held at appropriate positions in the guide grooves 56a to 56d of the main-unit-side housing 52, to thereby cause them to abut each other at the step portions 15A, 15B.
In the present embodiment, the stop positions of the first engagement members 19a to 19d are forcefully determined by the positions at which the first engagement members 19a to 19d respectively abut the second engagement members 57a to 57d that are threaded respectively around the threaded screws 59a to 59d. Therefore, neutral positions of the first motor M1 and the second motor M2 are preset. Thereby, when the insertion-portion-side housing 12 is replaced, positioning of the first engagement members 19a to 19d in accordance with optional positions of the second engagement members 57a to 57d is automatically performed. Hence, the straight position (neutral position) of the bending portion 9 can be automatically set.

Also with the aforementioned operation apparatus of the endoscope apparatus 50 according to the second embodiment, only an attachment of an integrally-coupled unit of an insertion portion 2 and an insertion-portion-side housing 12 to the main-unit-side housing 52 completes a replacement with one of another type, and enables an observation, an image-pickup or the like. In its replacement, even if the second engagement members 57a to 57d provided in the main-unit-side housing 52 are at their optional positions, abutment positions of the first engagement members 19a to 19d are determined in accordance with the positions of the second engagement members 57a to 57d. This makes it possible to bendingly hold the bending portion 9 at the bend angle in accordance with the abutment positions. Then, if the motors M1, M2 are driven to their neutral positions, the insertion portion 2 can be automatically adjusted to its straight, neutral position.
Therefore, in attaching the insertion-portion-side housing 12 for replacement, the positions of the first engagement members 19a to 19d and the second engagement members 57a to 57d can be automatically adjusted without the need to position them.
Furthermore, there is no need to install an encoder in the insertion-portion-side housing 12 as a replacement part to be removed. This makes the construction of the insertion-portion-side housing 12 simple, which also leads to a lower cost.

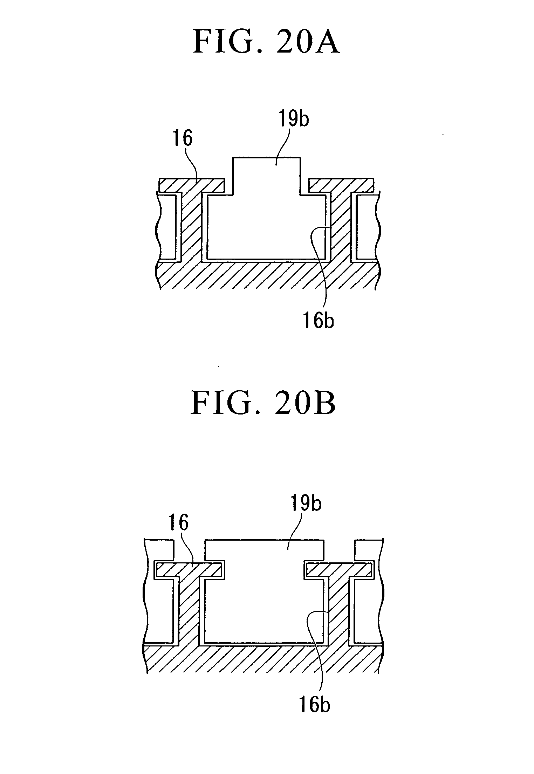

The aforementioned first embodiment has a construction in which the first and second engagement members 19a to 19d, 27a to 27d are connected to the first and second biasing members such as the coil springs 24a to 24d, 31a to 31d. The second embodiment has a construction in which the first engagement members 19a to 19d are connected to the coil springs 24a to 24d. However, the coil springs 24a to 24d, 31a to 31d need not necessarily be provided so long as the first and second engagement members 19a to 19d, 27a to 27d are configured to be held slidably in the forward-backward direction. Also in this case, with the first engagement members 19a to 19d and the second engagement members 27a to 27d being held in an engaged state, it is possible to perform an operation similar to those in the aforementioned embodiments.
For example, a construction in which the first engagement members 19a to 19d and the second engagement members 27a to 27d are held inseparable from the guide grooves 16a to 16d, 26a to 26d as shown in FIG. 20A or FIG. 20B may be adopted. Alternatively, the first and second engagement members may be held slidably in the guide grooves by magnetic attraction forces or rollers.

Furthermore, the wires 20a to 20d need not be fixed in the first engagement members 19a to 19b. It is permissible only if interlocking members of some kind are provided so as to allow a force to be transmitted between the wires 20a 20d and the first engagement members 19a to 19b. The same is true of the relationship between the operation wires 29a to 29d and the second engagement members 27a to 27d.
In the aforementioned embodiments, four sets of: the wires 20a to 20d and the first engagement members 19a to 19b; and the operation wires 29a to 29d and the second engagement members 27a to 27d are provided, to thereby allow the insertion portion 2 to be bendingly operated in the up, down, left, and right directions. However, the present invention is not limited to such a construction. For example, a single set of: a wire and an operation wire; and first and second engagement members may be provided, to thereby allow the insertion portion 2 to bend only in a single direction. Alternatively, more than one set of them may be appropriately provided, to thereby allow the insertion portion 2 to bend in more than one direction. Furthermore, with respect to the wires 20a to 20d and the first engagement members 19a to 19b, and the operation wires 29a to 29d and the second engagement members 27a to 27d, only one set for the up-down direction or the left-right direction may be provided, or two or more sets for the up-down direction and the left-right direction.
Furthermore, in the aforementioned first embodiment, the main-unit-side housing 13 is provided in the operation portion 3. In the aforementioned second embodiment, the main-unit-side housing 52 is integrally embedded in the controller 51. However, in the "second portion" of the present invention, the structure of a "housing" as distinguished from other members constituting the endoscope apparatus is not essential. That is, the specific shape, installation position, and the like of the "second portion" of the present invention are not particularly limited so long as they can have a desired function as the "second portion."
Furthermore, the operation apparatus of the insertion portion according to the present invention is not limited to an endoscope but is applicable to other treatment tools and the like.

### INDUSTRIAL APPLICABILITY

As described above, according to the present invention, in an endoscope apparatus for bendingly actuating an insertion portion of an endoscope, other treatment tools, or the like, it is possible to replace the insertion portion with ease without performing its positioning or the like.

## Claims

1. An endoscope apparatus, comprising:
a first portion that communicates with an insertion portion having a bendable bending portion;
a second portion that is detachably attached to the first portion; and
a drive portion that is provided on the second portion side and bendingly moves the bending portion,
wherein the first portion comprises:
a first pulling member that is connected to the bending portion and bendingly moves the bending portion; and
a first engagement member that is connected to the first pulling member and is capable of moving forward and backward in a longitudinal direction of the first pulling member,
wherein the second portion comprises:
an actuation member that is connected to the drive portion; and
a second engagement member that is connected to the actuation member, and is capable of moving forward and backward in the longitudinal direction of the first pulling member and of engaging the first engagement member, and
wherein engagement between the first engagement member and the second engagement member transmits an actuation of the drive portion to the bending portion of the insertion portion.

2. The endoscope apparatus according to claim 1, further comprising
a positioning-fixation mechanism that positions and fixes the first portion and the second portion, wherein
positioning and fixing the first portion and the second portion by the positioning-fixation mechanism engages the first engagement member with the second engagement member.

3. The endoscope apparatus according to claim 1, further comprising one or more wires as the first pulling member.

4. The endoscope apparatus according to claim 1, further comprising
one or more pairs of wires that move in mutually opposite directions as the first pulling member.

5. The endoscope apparatus according to claim 1,
wherein the first portion comprises a first biasing member that connects the first engagement member to a side opposite to the first pulling member in a biased state, and
wherein the second portion comprises a second biasing member that connects the second engagement member to a side opposite to the actuation member in a biased state, the actuation member being a second pulling member.

6. The endoscope apparatus according to claim 1, wherein the actuation member is a second pulling member, and the drive portion is an operation lever to which the second pulling member is connected.

7. The endoscope apparatus according to claim 1,
wherein the first portion comprises a first biasing member that connects the first engagement member to a side opposite to the first pulling member in a biased state, and
wherein the actuation member is a direct drive mechanism that moves the second engagement member forward and backward in the longitudinal direction.

8. The endoscope apparatus according to claim 7, wherein
the drive portion is a motor that rotationally actuates the direct drive mechanism to interlockedly move the second engagement member.

9. The endoscope apparatus according to claim 1, wherein
the first engagement member and the second engagement member each comprise one or more step portions, the step portions are capable of engaging and detaching from each other, and at least either one of the step portions being slidable along a guide groove.

10. The endoscope apparatus according to claim 1,
wherein a guide member is provided in one of the first portion and the second portion, and an anchor groove with which the guide member is engageable is provided in an other, and
wherein the first engagement member and the second engagement member are brought into engagement with each other in a state with the guide member in engagement with the anchor groove.

11. The endoscope apparatus according to claim 2, wherein the positioning-fixation mechanism further comprises: a lock member that fixes the first portion and the second portion in a coupled state; and a release member that releases locking by the lock member.

12. An endoscope apparatus that comprises an insertion portion having an observation system and an illumination system, comprising:
a first portion that communicates with an insertion portion having a bendable bending portion;
a second portion that is detachably attached to the first portion; and
an operation device that is provided in the second portion and bendingly moves the bending portion,
wherein the first portion comprises:
a first pulling member that is connected to the bending portion and bendingly moves the bending portion; and
a first engagement member that is connected to the first pulling member and is capable of moving forward and backward in a longitudinal direction of the first pulling member,
wherein the second portion comprises:
a second pulling member that is connected to the operation device; and
a second engagement member that is connected to the second pulling member, and is capable of moving forward and backward in a longitudinal direction of the second pulling member and of engaging the first engagement member, and
wherein engagement between the first engagement member and the second engagement member as a result of coupling the first portion to the second portion transmits an actuation of the operation device to the bending portion of the insertion portion.

13. An endoscope apparatus that comprises an insertion portion having an observation system and an illumination system, comprising:
a first portion that communicates with an insertion portion having a bendable bending portion;
a second portion that is detachably attached to the first portion; and
a drive source that is provided on the second portion side and bendingly moves the bending portion,
wherein the first portion comprises:
a first pulling member that is connected to the bending portion and bendingly moves the bending portion; and
a first engagement member that is connected to the first pulling member and is capable of moving forward and backward in a longitudinal direction of the first pulling member,
wherein the second portion comprises:
a direct drive mechanism that is connected to the drive source; and
a second engagement member that, in response to the direct drive mechanism, is capable of moving forward and backward in the longitudinal direction of the direct drive mechanism and is capable of engaging the first engagement member, and
wherein engagement between the first engagement member and the second engagement member as a result of coupling the first portion to the second portion transmits a drive of the drive source to the bending portion of the insertion portion.

14. The endoscope apparatus according to claim 13, wherein
the drive source is connected to an operation device for actuating the drive source.
